# EUROPEAN PATENT SPECIFICATION

(11) **EP 0 162 078 B1**
(45) Date of publication and mention of the grant of the patent: **14.09.1994**
(21) Application number: 84904241.1
(22) Date of filing: 29.10.1984
(51) Int. Cl.: C07K 17/02, A61K 39/395

(54) **METHOD FOR ISOLATING A PROTEIN FROM A MIXTURE CONTAINING SAID PROTEIN AND A CONFORMATION SPECIFIC ANTIBODY USEFUL THEREIN**
VERFAHREN ZUR ISOLIERUNG EINES PROTEINS AUS EINER DIESES PROTEIN ENTHALTENDEN MISCHUNG UNTER VERWENDUNG EINES KONFORMATIONSSPEZIFISCHEN ANTIKöRPERS
PROCEDE D'ISOLATION D'UNE PROTEINE D'UN MELANGE LA CONTENANT ET UN ANTICORPS SPECIFIQUE DE CONFORMATION

(30) Priority: 28.10.1983 US 546364
(43) Date of publication of application: 27.11.1985
(73) Proprietor: NEW ENGLAND MEDICAL CENTER HOSPITALS, INC., Boston, MA 02111 (US)
(72) Inventor: FURIE, Bruce, E., Wellesley, MA 02181 (US); FURIE, Barbara, C., Wellesley, MA 02181 (US); LIEBMAN, Howard, A., Newton, MA 02161 (US); LEWIS, Richard, M., Leesburg, VA 22075 (US)
(74) Representative: Deans, Michael John Percy
(86) International application number: US8401746
(87) International publication number: WO8501941

(56) References cited:
- US-A- 4 000 098
- US-A- 4 307 071
- US-A- 4 361 509
- US-A- 4 386 025
- US-A- 4 454 106
- US-A- 4 472 509
- BIOLOGICAL ABSTRACTS, vol. 66, 1978, ref. no. 37563, L. HOLMBERG et al.:"Purification of F.VIII:C by antigen-antibody chromatography" & THROMB RES12(4): 667-676. 1978
- PROC. NATL. ACAD. SCI USA, vol. 79, March 1982, pages 1648-1652;C.A. FULCHER etal.: "Characterization of the human factor VIII procoagulant protein with a heterologous precipitating antibody"
- TAI ET AL J OF BIOL CHEM 255-2790-2795 (1980)

## Description

The present invention is concerned generally with methods for the isolation of proteins, e.g., blood clotting proteins, from a mixture of proteins in a fluid, and with antibodies useful in such methods.

Proteins are commonly purified by immunoaffinity chromatography, in which a protein-containing mixture is contacted with an immobilized antibody to the protein, and this protein is then eluted under conditions, which in the conventional arrangement are non-specific and harsh, to disrupt the protein-antibody complex.

One class of proteins for which immunoaffinity has been attempted are the proteins involved in the blood clotting process. The general overall process of blood clotting involves two stages: an activation stage in which the proenzyme prothrombin, through the action of many Factors and calcium ions, is converted into its active enzyme form, thrombin, and a conversion stage in which the proteolytic enzyme thrombin acts upon fibrinogen to form fibrin, which forms a three dimensional network mesh that holds the formed elements of blood.

The requisite Factors for blood clotting are all proteins, several of which share some similarities in structure and function, while others are distinct moieties unlike any other. For example, four blood clotting proteins (the "vitamin K-dependent proteins") require vitamin K for their complete synthesis: Factor IX, Factor X, Factor VII, and prothrombin. As a group, these proteins share marked homology in amino acid sequence, are activated by limited proteolysis from the zymogen to active enzyme form, and contain the novel metal binding amino acid γ-carboxyglutamic acid. These blood clotting proteins are representative of a unique class of metal-ion binding proteins which are able to bind a large number of bivalent and trivalent cations. Upon combination with metal-ion, such as calcium, magnesium, manganese and gadolinium ions, these proteins undergo a structural conformational transition involving changes in the peptide backbone and changes in exposure of specific amino acid residues, which can be monitored by fluorescence, circular dichroism, or immunochemical techniques.

Other blood clotting proteins also share this ability to bind with metal-ions. Factor V, proaccelerin, is essential in the conversion of prothrombin to thrombin and is a very labile protein which rapidly disappears from stored plasma. Factor VIII, antihemophilic factor, is essential for the making of thrombin and is deficient in the plasma of persons with classical hemophilia. Hemophilia is congenital and the blood of hemophiliacs appears normal relative to the coagulation mechanism except for the deficiency of Factor VIII.

The vitamin K dependent proteins are deficient, on an acquired basis, in liver disease, in vitamin K deficiencies and in the presence of vitamin K antagonist drugs such as sodium warfarin (Coumadin). Hemophilia B is a disorder characterized as a hereditary deficiency of Factor IX; of the 25,000 persons in the United States with hemophilia, approximately 10-12% are afflicted with Hemophilia B.

The treatment of persons whose disorders comprise acquired or congenital deficiencies of blood clotting proteins continues to be a high risk and costly therapy. For example, Hemophilia B is presently treated in two ways: use of fresh frozen plasma or use of a commercial preparation of Factor IX. This latter material is a concentrate obtained by partial fractionation of normal human plasma and is, at best, only of intermediate purity. Both therapies, the frozen plasma and the impure Factor IX concentrate, present a significant risk of hepatitis to the patient, but the Factor IX concentrate presents a far greater risk of infection since it is prepared from pooled human plasma. Essentially all hemophiliacs receiving multiple transfusions of either of these plasma products have been exposed to hepatitis and show seriological evidence of such exposure. Clinically, most have some form of abnormal liver function. However, the impure Factor IX concentrate adds risk of major complications, such as disseminated intravascular coagulation, thrombosis, and hepatitis, among others, believed to be directly caused or aggravated by the impurities in the preparations. More recently, an increased risk for the development of the highly fatal Acquired Immune Deficiency Syndrome (AIDS) has been reported in patients with hemophilia who received plasma concentrates. Although plasma protein infusion therapy is still the treatment of choice in these disorders, it is clear the complications of such infusion therapy, caused directly by the impurities in the prepared product, diminish its use and effectiveness. For this reason, any method which would provide blood clotting plasma protein in concentrated form of substantial purity would eliminate or significanlty reduce the undesirable medical complications of current infusion therapy. Such an advance would satisfy a long recognized need and provide additional advantages such that infusion therapy may be used regularly and prophylactically by hemophiliacs to reduce or eliminate the protein deficiencies associated with such disorders.

As mentioned above, general procedures are known for purifying blood clotting factors in plasma by passing the plasma through an affinity chromatographic column comprising inert matrix support, usually in the form of beads, such as Sepharose to which is bound the antibody to the factor it is desired to isolate. The factor specifically complexes with the fixed antibody and thereafter the factor (antigen) is eluted from the column. However, prior to this invention, it has been very difficult to obtain therapeutically useful purifications of the desired blood clotting factors by this process since the blood clotting factors are very difficult to elute successfully. This is because the chemical or physical conditions necessary to separate the antibody from the protein can destroy the function of the protein.

Accordingly, it would be highly desirable to provide a means for isolating proteins including individual blood clotting factors whereby both the structural and functional integrity of the protein can be retained and whereby the proteins can be recovered in quantity.

As we shall explain below, the invention enables us to provide highly effective practical methods for isolating proteins which undergo conformational changes (i.e., a change in tertiary structure) when complexed with ligands. The methods employ antibodies (either polyclonal or monoclonal) which specifically react with protein-ligand complexes, and substantially fail to react with the protein in the absence of the ligand. (Since the protein is generally stabilized by the ligand, the protein, complexed with the ligand, is sometimes referred to herein as a "ligand-stabilized" conformer).

As explained in more detail below, our practical methods involve immobilizing the antibody on a solid support and then contacting a mixture containing the protein with the immobilized antibody, in the presence of the ligand, to bind the ligand-stabilized protein to the immobilized antibody. To release the protein, the protein-antibody complex is contacted with a compound having a binding affinity for the ligand higher than the affinity of the protein for the ligand; this higher affinity compound removes the ligand from the protein, changing the protein's conformation so that the antibody no longer binds to it, and the protein is thus released. This releasing step is specific and is carried out under mild conditions, and thus provides a high degree of purification without the risk of denaturation and loss of function associated with the non-specific, harsh conditions under which proteins are conventionally eluted from immunoaffinity columns.

Accordingly, there is provided, in accordance with a first aspect of the present invention, a conformation specific antibody immobilized on a solid support, said antibody immobilized on said support being reactive with a free protein complexed with a ligand and substantially unreactive with said free protein not complexed with said ligand.

The invention provides, in a second and alternative aspect thereof, a method for isolating a protein from a mixture containing said protein, said method comprising providing a conformation specific antibody immobilized on a solid support, said antibody being reactive with said protein complexed with a ligand and substantially unreactive with said protein not complexed with said ligand, contacting said mixture, in the presence of said ligand, with said immobilized antibody to bind said protein, complexed with said ligand, to said immobilized antibody to form an immune complex, and contacting said immune complex with a compound having a binding affinity for said ligand higher than the binding affinity of said protein for said ligand, to release said protein from said immobilized antibody.

Our methods provide an additional very important advantage: proteins, e.g., proteins involved in human blood coagulation, are separated not only from other proteins in the mixture, but from viral contaminants as well. This is extremely important for haemophiliacs, who frequently contract hepatitis B from conventional Factor VIII and Factor IX preparations.

In addition, the methods of the invention provide high purification in a few simple steps, and are susceptible to inexpensive automation and scale-up.

Other features and advantages of the invention will be apparent from the following description of preferred embodiments thereof.

The drawings will first be described.

Fig. 1 is a graph illustrating the binding of conformation specific anti-Factor IX polyclonal rabbit antibodies to Factor IX in calcium chloride (·) and in ethylenediaminetetraacetic acid (o).

Fig. 2 is a graph illustrating the direct binding of conformation specific anti-Factor IX monoclonal antibodies (HL12-21) to Factor IX in the presence and absence of calcium chloride.

Fig. 3 is a graph illustrating the specificity of conformation specific anti-Factor IX: Ca⁺⁺ polyclonal antibodies. Displacement is observed with Factor IX (·) , but not with Factor X ( ▲ ) or prothrombin (o).

Fig. 4 is a graph illustrating the purification and isolation of human Factor IX from partially purified plasma using a method of the invention.

Fig. 5 is a graph illustrating the binding of conformation specific anti-prothrombin monoclonal antibody to prothrombin in the presence of calcium chloride and its elution with ethylenediaminetetraacetic acid.

Fig. 6 is a graph illustrating the removal of Hepatitis B surface antigen from Hepatitis B-contaminated plasma, using a method of the invention. The column fractions were assayed for protein concentration (absorption 280 nm ) and hepatitis B surface antigen (HBsAg ) by competition radioimmunoassay. The quantitative presence of hepatitis virus is expressed as an increase in cpm in the RIA (non-specific binding is 124 cpm, positive samples greater than 250 cpm).

The method of the invention can be used to isolate any protein whose conformation is changed when the protein is complexed with a ligand.

One class of such proteins are the human and other mammalian plasma proteins involved in blood coagulation, i.e., Factors V, VII, VIII, IX, X, prothrombin, protein S, and protein C. These proteins undergo a conformational change when complexed with certain low molecular weight
( <3,000) ligands, particularly divalent or trivalent metal ions such as Ca⁺⁺, Mg⁺⁺, Mn⁺⁺, Co⁺⁺, and Gd⁺⁺. Some of these proteins, e.g., prothrombin and Factor IX, have a metal ion binding site containing γ-carboxyglutamic acid, while others, e.g., Factor V do not contain γ-carboxyglutamic acid, but nonetheless undergo a conformational change when complexed with a metal ion.

Other proteins which can be purified using methods in accordance with the invention are enzymes which can complex with a ligand to form a stable complex whose three-dimensional structure is different from that of the uncomplexed enzyme. Examples of such ligands are very poor substrates; suicide substrates (i.e., those substrates which are activated by the enzyme and form a covalent complex with the enzyme); substrate analogs which function as inhibitors; and enzyme inhibitors.

### Antibody Production

The antibodies used in our methods can be either polyclonal or monoclonal antibodies, produced by conventional techniques. As the antibody is to be specific for the ligand-stabilized conformer, immunization is carried out using the ligand-stabilized conformer of the protein or, if the ligand is already present in the immunized animal, the protein alone, without the ligand, can be used; the ligand present in the animal complexes with the immunizing protein in vivo, resulting in the production of antibodies to the ligand-stabilized conformer. For example, since calcium is present in animal blood, antibodies to Ca⁺⁺-stabilized human blood proteins such as Factor IX can be generated by immunizing with Factor IX not complexed with Ca⁺⁺. On the other hand, if the ligand is one not present in the serum of the immunized animal, e.g., a substrate for an enzyme, immunization is carried out using the ligand-stabilized protein.

### Antibody Screening and Purification

Antibodies are screened for the ability to bind to the protein complexed with ligand and not to protein not so complexed, using conventional screening techniques. Antibodies are purified using affinity columns to which is bound the ligand-complexed-protein; elution is carried out with a compound having high affinity for the ligand.

### Binding to a Solid Support

The purified antibody is then bound to any conventional solid support used in protein purification techniques, e.g., an affinity chromatography column to which crosslinked agarose, polyacrylamides, or cellulose is attached via, e.g., cyanogen bromide, carbodiimide, or protein A. Conventional solid supports, e.g., various polymeric beads, used in non-chromatographic affinity purification methods can be used as well.

### Protein Isolation

Proteins are isolated by contacting a protein-containing mixture with the appropriate support-bound antibody, in the presence of the appropriate ligand. Disruption of the immune complex is then achieved by changing the conformation of the bound protein by removing the ligand. Where the bound protein is metal ion-stabilized, disruption is preferably achieved using a metal chelator such as EDTA, EGTA, citrate, oxalate, or phosphate.

The following specific examples are intended to more particularly point out the present invention, without acting as limitations on its scope.

### Example 1

### PREPARATION OF PURIFIED HUMAN FACTOR IX CONCENTRATE

### Preparation of Human Factor IX Antigen

Factor IX was isolated from fresh frozen human plasma by sequential barium citrate adsorption and elution, DEAE Sephadex chromatography, DEAE cellulose chromatography and heparin-Sepharose chromatography, according to the methods described in Rosenberg et al., (1974) J. Biol. Chem., 250:1607-1617; and Miletich et al., (1978) J. Biol. Chem., 253: 6908-6916. The purified Factor IX migrated as a single band upon electrophoresis in polyacrylamide gels with dodecyl sulfate. Factor IX activity was determined with a two stage assay using Factor IX-deficient plasma, and was shown to have a specific activity of 180-200 units/mg.

Purified Factor IX was coupled to cyanogen bromide-activated Sepharose 4B at a concentration of 4.3 mg per ml of Sepharose (total volume 4 ml Sepharose).

### Preparation of anti-Factor IX:Ca⁺⁺ Antibodies

New Zealand white rabbits were immunized with Factor IX. Antibodies specific for the metal-stabilized conformation of Factor IX (anti-Factor IX:Ca⁺⁺) were purified by affinity chromatography on the human Factor IX-Sepharose column (1.5 x 3 cm) as a modification of the technique of Tai et al. (1980) J. Biol Chem., 225:2790-2795, as follows. Antiserum was dialyzed overnight in 0.05 M Tris HCl, pH 7.4, 0.14 M NaCl, 3 mM CaCl₂, 0.05% NaN₃ (TBS/CaCl₂). The Factor IX-Sepharose column was equilibrated with the same buffer and the antisera was applied to the column. The column was exhaustively washed with the TBS/CaCl₂ to remove unbound protein. The anti-Factor IX:Ca⁺⁺ antibodies were eluted with TBS/5 mM EDTA. The bound anti-Factor IX antibodies which bind to Factor IX in the absence of metal ions were eluted with 4 M guanidine HCl.

The antibodies eluted with 5 mM EDTA (those specific for the Ca⁺⁺-stabilized conformer) were pooled and concentrated by ultrafiltration; these represented approximately 20% of the antibodies in the antiserum. Rabbit anti-Factor IX:Ca⁺⁺ antibody was coupled to cyanogen bromide-activated Sepharose 4B at a concentration of 3.3 mg per ml Sepharose (total volume 2 ml Sepharose) according to the method of Cuatrecasas et al. (1969) PNAS USA, 61:636-643.

### Preparation of Monoclonal Conformation Specific Anti-Factor IX Antibodies

Balb/c mice were immunized with an initial peritoneal injection comprising 50 µg of human Factor IX antigen in complete Freund's adjuvant. These mice were then immunized biweekly with 25 µg of Factor IX in complete Freund's adjuvant for three months. Following a one month time period without any further immunization, these mice were injected with 25 µg of Factor IX in 0.15 M NaCl solution intravenously for the next three consecutive days prior to cell fusion.

Spleen cells (approximately 5 x 10⁷ cells) from immunized mice were fused with the Sp2/0 plasma cell line (5 x 10⁶ cells in 28% polyethylene glycol 5000, Sigma Corporation) using the method of Kohler and Milstein [Kohler, G. et al., Nature (London), 256:495-497 (1975)]. Fused cells were suspended in complete medium comprising RPMI 1640, 15% Donor calf serum, 10 mM Hepes buffer, 4 mM glutamine and 20 g/ml gentamycin and grown in this media for 48 hours. Fused cells were then removed from complete medium and resuspended in hypoxanthine-aminopterin-thymidine (hereinafter HAT) containing growth medium. The cell suspension was then distributed into individual dual wells of a microtiter tray as aliquots containing approximately 3 x 10⁵ cells per well for continued cell growth. Supernatants from each well were assayed for anti-Factor IX antibody production after several weeks. Selected cell cultures were cloned by the limiting dilution method [McKearn, T.J. et al., Monoclonal Antibodies, Plenum Press, New York]. Although many clones were identified that produced monoclonal antibodies reactive to Factor IX, a single clone (designated HL 12-21) produced conformation-specific antibodies reactive with Factor IX only in the presence of metal-ions and not reactive with Factor IX in the absence of metal ions.

### Assay for Evaluation of Anti-Factor IX Antibody

A solid phase enzyme linked immunoabsorbent assay (ELISA) method was used for evaluating polyclonal rabbit and monoclonal murine anti-Factor IX:Ca⁺⁺ antibodies. An appropriate number of wells in microtiter plates were coated with human Factor IX at 20µ g/ml concentration in 0.05 M borate (pH 8.5) for sixteen hours at 4^{o}C. The plates were exhaustively washed with Buffer A comprising 50 mM Tris HCl (pH 7.2), 0.14 N NaCl, and 0.05% NaN₃ and the buffer A containing 2% bovine serum albumin was added to the wells for thirty minutes at 24^{o}C. After an extensive washing with Buffer A alone, 50 ul of tissue culture supernatant or polyclonal anti-Factor IX anti-serum was added to each respective well and then the plates incubated at 37^{o}C for one hour. Each well was then extensively washed with Buffer B comprising 50 mM Tris-HCl (pH 7.2), 0.14 N NaCl, 1.5 mM MgCl₂, 2 mM beta-mercapthoethanol, 0.05% NaN₃ and 0.05% Tween 20. Fab fragments of antimouse Ig (50 µl) raised in sheep were conjugated to beta-galactosidase in Buffer B and then added to each well. After the plates were again incubated for two hours at 24^{o}C, they were washed with Buffer B three more times. An enzyme substrate comprising p-nitrophenyl D-galactoside (50 µl in 0.05 M sodium phosphate, pH 7.2), 1.5 mM MgCl₂ and 100 mM beta-mercaptoethanol was added to each well and the reaction permitted to proceed for between thirty to sixty minutes at 24^{o}C. The reaction product was monitored by measuring the absorbance at 405 nanometer (hereinafter nm) using a Dynatech MR 580 micro-ELISA autoreader.

For those studies evaluating the effect of calcium ions on antibody-Factor IX interaction, an additional step in the ELISA procedure was included. After incubation of monoclonal antibody with Factor IX coated wells, the plates were washed with a buffer comprising 50 mM Tris-HCl and 0.14 M NaCl, pH 7.2 containing either 10 mM EDTA or 5 mM CaCl₂. After two washings with the CaCl₂ or EDTA containing buffer, bound mouse immunoglobulin was detected and quantitated as described above.

In addition, in those experiments using polyclonal rabbit antibodies, anti-rabbit Ig (50 l) raised in sheep was conjugated to alkaline phosphate in Buffer B without β mercaptoethanol and this fluid added to the appropriate wells followed by incubation at 24^{o}C for two hours. After washing the wells in Buffer B, ρ-nitrophenyl phosphate disodium (50 µl in 1 M glycine, 1.5 mM MgCl₂, pH 10, were added to each well and the reaction was allowed to proceed for sixty minutes at 24^{o}C and stopped with 3 N NaOH. The reaction product was monitored and measured by absorbance at 405 nm.

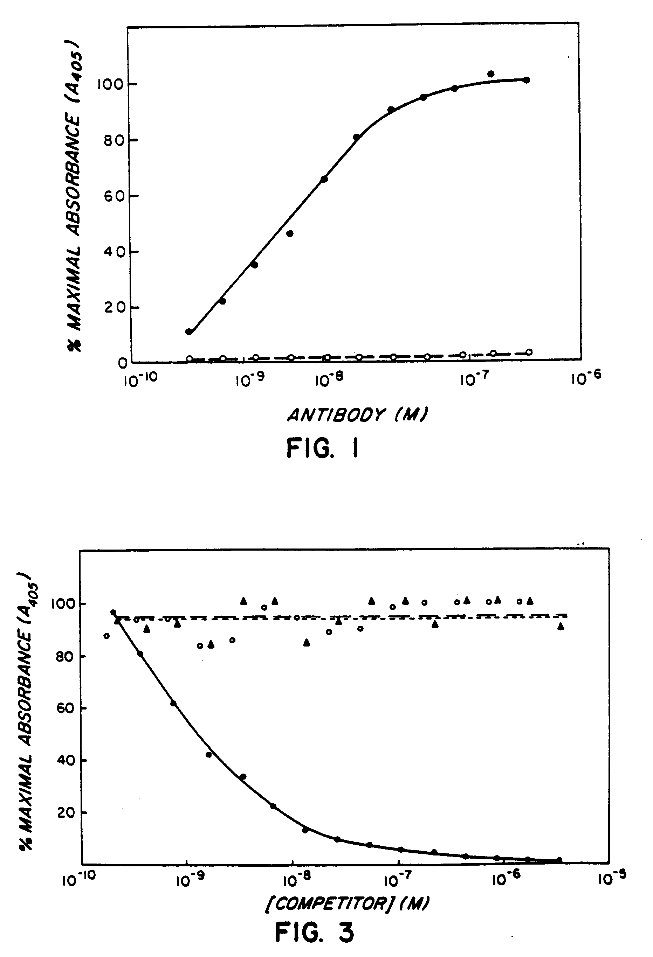

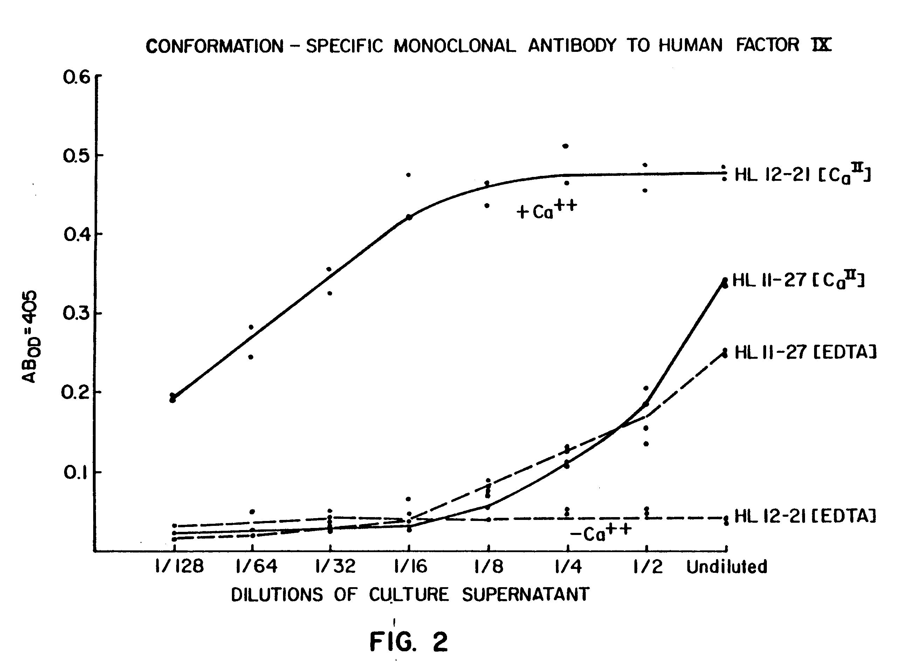

The results of evaluating polyclonal and monoclonal conformation specific anti-Factor IX:Ca⁺⁺ antibodies are illustrated graphically in Figs 1 and 2. Fig. 1 demonstrates the binding capability of rabbit anti-Factor IX:Ca⁺⁺ polyclonal antibodies in the presence of either CaCl₂ or EDTA as is apparent therein, the ability of these conformation specific antibodies to bind with Factor IX antigen is substantially reduced in the presence of EDTA. Fig. 2 illustrates the direct binding of HL 12-21 murine monoclonal antibody to Factor IX antigen in sequential dilution in the presence of calcium ion or EDTA. Specifically, one antibody clone, HL 12-21, reveals the inability of the conformation specific monoclonal antibody to bind to Factor IX antigen in the presence of EDTA.

### Evaluation of Antibody Specificity for Anti-Factor IX:Ca⁺⁺

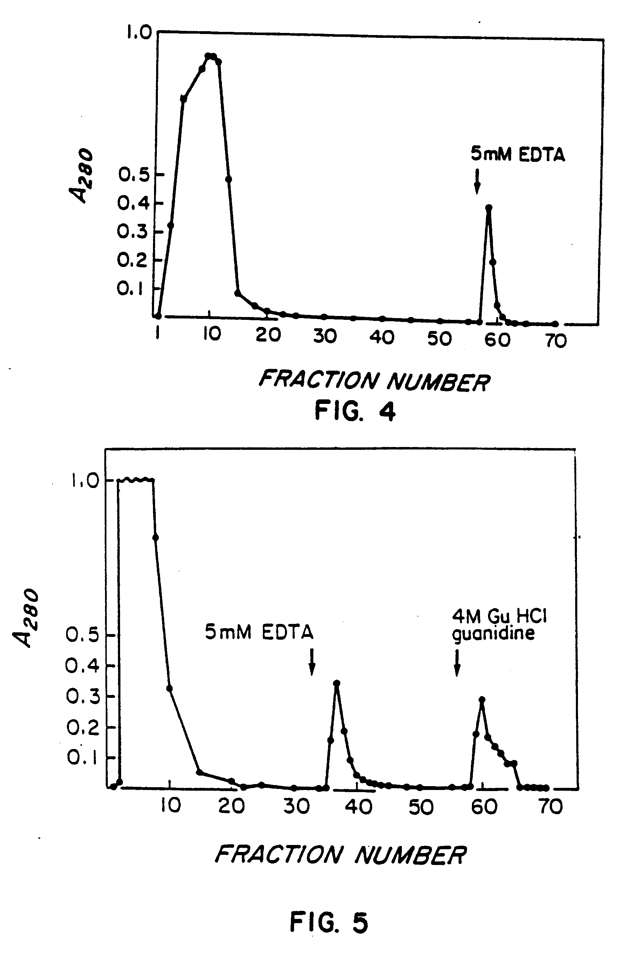

The determination of antigenic specificity for conformation specific anti-Factor IX antigen murine monoclonal and rabbit polyclonal antibodies utilized two types of assays. The first assay employed a microtiter plate whose wells were coated with either 20 ug/ml of human prothrombin, Factor X, or Factor IX which were then combined and allowed to react with the conformation specific antibodies. It was found that all of the antibody populations under test, the murine monclonal conformation specific antibodies and the polyclonal rabbit conformation specific antibodies, reacted with and bound to the human Factor IX antigen exclusively. In the second assay (Fig. 3), human prothrombin, Factor X and Factor IX were added individually to separate wells at varying concentrations to a constant amount of murine monoclonal or rabbit polyclonal conformation specific antibody. Following an initial reaction time of 30 minutes, the reaction fluids from those wells comprising prothrombin, Factor IX or Factor X were subsequentaly added to other microtiter wells coated with Factor IX antigen. The interaction of the initial reaction fluids containing conformation specific anti-Factor IX:Ca⁺⁺ antibody with the other plasma proteins instead of immobilized Factor IX antigen was monitored as a decrease in the amount of immunoglobulin which bound to the immobilized solid phase containing Factor IX. The results are graphically illustrated in Fig. 3 in which rabbit polyclonal anti-Factor IX:Ca⁺⁺ antibodies competed poorly, if at all (less than 10,000 x) with human prothrombin or Factor X was conclusively demonstrating the specificity of these conformation specific antibodies for Factor IX in the precence of calcium ions.

### Isolation of Purified Human Factor IX Using a Conformation Specific Anti-factor IX Antibody-Sepharose Matrix

The binding specificity of conformation specific polyclonal or monoclonal anti-Factor IX was demonstrated by the application of purified human Factor IX, Factor X or prothrombin to an affinity matrix comprising murine monoclonal anti-Factor IX:Ca⁺⁺ antibodies or rabbit polyclonal anti-Factor IX:Ca⁺⁺ antibodies coupled to cyanogen bromide-activated Sepharose using established methods [Cuatrecasas, P. et al., Proc. Natl. Acad. Sci. USA, 61:636 (1969)]. Purified preparations of these vitamin K dependent coagulation proteins were dialyzed against Buffer A containing 1 mM CaCl₂ and 1 mM benzamidine (pH 7.5) and then applied to an affinity matrix column equilibrated with this dialysate. The Factor IX protein bound to the affinity matrix while the Factor X and prothrombin proteins were eluted by the dialysate fluid. Subsequently, the Factor IX protein binding to the affinity matrix was eluted with Buffer A containing 1 mM benzamidine (pH 7.5) and 3 mM EDTA. The recovered Factor IX protein was then dialyzed against 10 mM sodium phosphate, 0.14 N NaCl (pH 7.0) and then frozen at -70^{o}C. The separation and individual elution of the respective plasma proteins from the affinity matrix was monitored by measuring the absorption at 280 nm. The functional activity of the purified human Factor IX protein isolated from the affinity matrix was evaluated. by assay. In all cases, the purified Factor IX protein was found to be structurally intact and functionally active. In addition, the functional activities of Factor X and prothrombin recovered in the initial elution fluid through the affinity matrix column were also assayed by testing each protein's ability to accelerate the clotting of bovine Factor X-VII or Factor II-VII deficient plasma using the Russell's viper venom-cephalin coagulation procedure.

Another demonstration of the selective purification of human Factor IX from barium citrate absorbed plasma further purified with DEAE Sephacel or commercially prepared Proplex® concentrate was performed in the manner described above. The results of the protein fractions obtained using this eluent fluid is graphically illustrated in Fig. 4. The purity of the Factor IX protein fraction eluted by the fluid containing EDTA (Fig. 4) was evaluated by electrophoresis in polyacrylamide gel containing dodecyl sulphate and compared to electrophoretic gels of the partially purified plasma protein material applied to the affinity column and compared to the proteinaceous material eluted in flow-through in Fig. 4 which did not bind the column in the presence of CaCl₂ containing eluent. The specific activity of human Factor IX protein in the partially purified plasma protein material was 12.6 units/mg of protein. After isolation of human Factor IX protein from an affinity matrix column comprising rabbit polyclonal anti-Factor IX:Ca⁺⁺ antibody using eluent containing EDTA, the specific activity (post dialysis) of the Factor IX protein was 152 units/mg. This 13-fold increase in purity is equal to or more pure than the Factor IX protein obtained by previously known multi-step techniques. This method removes most, if not all, of the contaminating Factor X and prothrombin activity as well as other proteins. The results are shown in Table 1. For reasons that are not yet entirely clear, much better results were obtained using the polyclonal antibody than using the monclonal antibody.

The major advantage offered by this method of purification include the following: concentrated preparations of marked purity, having between 95-100% homogeneity, are obtainable consistently using an affinity matrix purification procedure which is considerably simpler than the classical multi-step purification techniques presently known; in comparison to the presently available products for plasma infusion therapy and the Konyne® or other commercial concentrate, a pure protein product from 20 to 12,000 times purer may be obtained. It will be appreciated by those skilled in this art that this methodology enlarged to commercial scale represents a major decrease in the cost of producing plasma infusion products and offers a superior product which will eliminate the undesirable medical complications presently accepted as a consequence of present plasma infusion therapy methods.

### Separation of Factor IX from Hepatitis B Virus

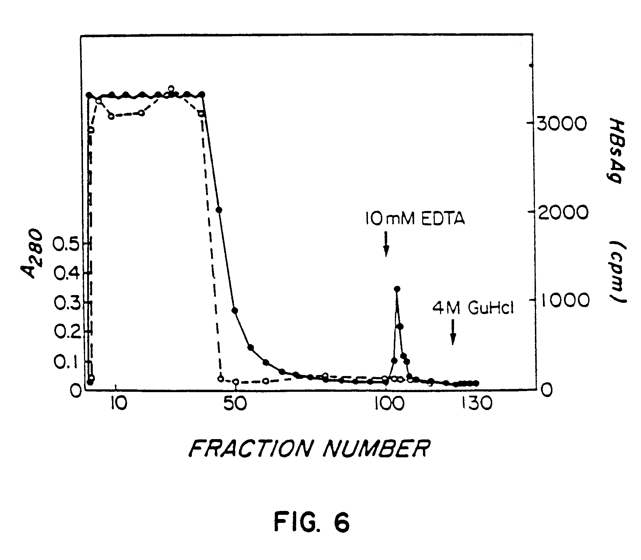

Partially purified Factor IX concentrates used in the treatment of hemophilia B are associated with a high risk of hepatitis virus contamination. We questioned whether the purified Factor IX prepared by immunoaffinity chromatography would be free of viral contaminants. Ascites (1 ml) from a patient with primary hepatocellular carcinoma, rich in hepatitis B virus as measured by the assay of hepatitis B surface antigen, was added to fresh frozen plasma (200 ml). The Factor IX was purified by immunoaffinity chromatography, as described above, using anti-Factor IX:Ca⁺⁺-Sepharose. Viral surface antigen was measured by radioimmunoassay in column fractions. All of the hepatitis B surface antigen failed to bind to the affinity matrix. There was no detectable hepatitis virus in the EDTA eluate containing Factor IX (Figure 6). After a 50-fold concentration of the Factor IX fractions no hepatitis virus was detectable. Within the limits of detection these results indicate that Factor IX is separated from hepatitis virus during its purification.

### Example 2

### PREPARATION OF PURIFIED HUMAN PROTHROMIN CONCENTRATE

Human prothrombin can be isolated using an immobilized antibody specific for the Ca⁺⁺-stabilized conformer of prothrombin. The antibody is made by immunization with prothrombin. As in the case of Factor IX purification, the first stage is the preparation of antigen for immunization

### Preparation of Prothrombin Antigen

Human prothrombin was prepared from fresh frozen plasma using established methods of protein precipitation used in sequence comprising: barium citrate adsorption, ammonium sulfate precipitation: ion exchange chromatography and dextran sulfate-agarose chromatography [Rosenberg, J.S. et al., J. Biol Chem., 250:1607-17 (1974) and Miletch, J.P. et al., J. Biol. Chem., 253:6908-6914 (1978)]. The purified prothrombin obtained in this manner was shown to have specific activity of 10 units/mg by coagulation assay (Fullerton, K.W., Lancet, 2:195 (1940).

### Preparation of Polyclonal and Monoclonal Conformation Specific Anti-Prothrombin Antibodies

Polyclonal conformation specific antibodies to prothrombin were raised by immunization of New Zealand white rabbits in the manner described above in Example 1. After collection of the blood by venopuncture and centrifugation, the serum fraction was used as the source of antibodies which were subsequently removed by affinity chromatography on a column matrix. The affinity matrix comprised prothrombin which had been covalently linked to cyanogen bromide-activated Sepharose using established methods. Anti-prothrombin:Ca⁺⁺ antibody was eluted from the affinity matrix using Buffer A containing EDTA.

The preparation of murine monoclonal conformation specific anti-prothrombin antibodies and the evaluation of such conformation specific antibodies generally using the ELISA methodology follow the respective descriptions for each represented within Example 1.

### The Evaluation of Monoclonal Antibody Specificity

The antigenic specificity of several monoclonal antibodies derived from those cloned identified as RL 1.3 and RL 1.9 were evaluated using a competitive assay based upon the ELISA methodology. Antibodies from these clones were shown to bind to immobilized human prothrombin; additional free prothrombin was then added which competed with the immobilized prothrombin for the antibodies. Using this competitive assay, the interaction of these monoclonal antibodies with prothrombin fragment 1, abnormal (des-γ carboxyglutamic acid) prothrombin, thrombin, prothrombin 1 and bovine prothrombin were examined. The results demonstrated that both types of monoclonal antibodies (RL 1.3 and RL 1.9) bound fragment 1 (the NH₂-terminal third portion of prothrombin) while neither of these bound prothrombin 1 (the COOH-terminal two third portion of prothrombin). These antibodies did not bind at all to thrombin (which had been previously treated with p-amidinophenylmethanesulfonyl fluoride). Significantly, higher concentrations (ranging from 2-fold to 10-fold) of prothrombin fragment 1 compared to prothrombin were required to inhibit 50% of antibody-imobilized prothrombin interaction. In addition, neither of these monoclonal antibody types bound to bovine prothrombin.

Monoclonal antibodies from three hybridoma culture supernatants were then examined for prothrombin binding activity in the presence and absence of calcium ions. The clones RL 1.3, RL 1.9, and HL 10.6 produced conformation specific antiprothrombin antibodies which bound prothrombin in the presence of 5 mM CaCl₂, but showed no significant binding in the presence of 0.01 M EDTA. Clone RL 1-3 has been deposited in the American Type Culture Collection (ATCC) and given ATCC Accession No. HB 8637.

### Purification of Conformation Specific Anti-Prothrombin Antibodies from a Monoclonal Antibody Pool

Hybrid clones producing a variety of anti-prothrombin antibodies were grown in large volumes of culture fluid using established methods [Lewis, R. et al., Biochemistry, 22:948-954 (1983)]. Several types of antibodies were purified from a 50% ammonium sulfate fraction of such fluids by affinity chromatography. The antibody pool was applied to a 2 x 6 cm column affinity matrix comprising prothrombin covalently bound to agarose which was previously equilibrated with an eluent comprising Tris-HCl (pH 7.5), 0.5 M NaCl and 5 mM CaCl₂ (Fig. 5). The eluted fraction obtained after passage through this affinity matrix were monitored by measuring the absorbance of the fractions at 280 nm. After the affinity matrix was washed free of unbound protein, the bound protein fraction was eluted using an eluent comprising 0.05 M Tris-HCl (pH 7.5), 0.5 M NaCl and 10 mM EDTA. The proteins in this eluent were then dialyzed against a dialysate comprising 0.05 M Tris-HCl (pH 7.5), 0.15 M NaCl and 0.02% sodium azide for 16 hours and subsequently stored at -20^{o}C.

### Interaction of Conformation Specific Anti-Prothrombin Antibodies with Prothrombin

The interaction of monoclonal antibodies obtained from the RL 1.3 clone with prothrombin was studied using a wide range of calcium ton concentrations using the ELISA method. To eliminate contaminating calcium ion as a source of potential error in the assay, microtiter plates containing immobilized prothrombin were washed with a buffer containing EDTA and then exhaustively washed with a fluid comprising 0.05 N Tris-HCl (pH 7.5) and 0.15 M NaCl prepared with metal-free water. The results demonstrated that all of the monoclonal antibody binding to the immobilized prothrombin was calcium dependent. Maximal binding was observed at a concentration of 0.9 mM CaCl₂ and half-maximal binding was observed at a concentration of 0.1 mM CaCl₂. Similarly, the binding of RL 1.3 antibodies to insolublized prothrombin was measured in which varying concentrations of RL 1.3 anti-prothrombin antibody was added to a microtiter plate whose wells were coated with excess prothrombin. Using the empirical data obtained, a Scatchard plot was prepared in which the binding constant of monoclonal anti-prothrombin conformation specific antibody, Kₐ, was calculated to be 2.3 x 10⁹M⁻¹. It was noted that the binding curve was linear over the entire concentration range evaluated indicating that a single population of antibody combining sites was involved as expected for the monoclonal conformation specific antibody preparation.

### Preparation of Monoclonal or Oligoclonal Affinity Matrices for Isolation of Purified Prothrombin

These monoclonal, conformation specific, anti-prothrombin antibodies are used to prepare an affinity matrix for the isolation of prothrombin protein which bind to calcium ions to form a calcium ion stabilized form of prothrombin. The methods used are similar to those described earlier in Example 1 regarding the use of polyclonal or monoclonal anti-Factor IX:Ca⁺⁺ affinity matrices. One major advantage of the hybridoma produced monoclonal anti-prothrombin antibodies is that distinctly different monoclonal antibody populations, each being conformation specific for the calcium ion stabilized form of prothrombin, can be combined in defined portions to form an oligoclonal antibody pool which provide optimum prothrombin binding capabilities with subsequent elution of the bound prothrombin as a purified molecule. This pool of oligoclonal antibodies is linked to cyanogen bromide-activated agarose in a manner identical to monoclonal or polyclonal antisera to form an affinity matrix. Prothrombin containing fluids or prepared fractions are then applied to the affinity matrix in the presence of 5 mM CaCl₂ following elution of the non-binding materials using calcium ion containing buffers; the affinity matrix is then washed with citrate buffer or an eluent containing 10 mM EDTA. The calcium ions in the metal-ion stabilized forms of prothrombin bound to the affinity matrix become preferentially bound to the citrate buffer or the EDTA, thus dissociating the prothrombin-anti-prothrombic complex on the surface of the affinity matrix. It will be appreciated that it is this preferential binding of the metallic cation, the calcium ion in this instance, to the EDTA or citrate buffer which causes the dissociation of the prothrombin-antibody complex and the concomitant dissociation of the metal-ion stabilized form of prothrombin concurrently. The mechanism of antigen-antibody complex dissociation regardless of the exact identity of the metallic ion used and regardless of the identity of the blood coagulating plasma protein isolated, is similar in all instances.

## Claims

1. A conformation specific antibody "to a free protein which undergoes conformational changes when complexed with a ligand" immobilized on a solid support, said antibody immobilized on said support being reactive with a free protein complexed with a ligand and substantially unreactive with said free protein not complexed with said ligand.

2. A method for isolating a protein from a mixture containing said protein, said method comprising
providing a conformation specific antibody immobilized on a solid support, said antibody being reactive with said protein complexed with a ligand and substantially unreactive with said protein not complexed with said ligand,
contacting said mixture, in the presence of said ligand, with said immobilized antibody to bind said protein, complexed with said ligand, to said immobilized antibody to form an immune complex, and
contacting said immune complex with a compound having a binding affinity for said ligand higher than the binding affinity of said protein for said ligand, to release said protein from said immobilized antibody.

3. The method of claim 2 wherein said protein is a protein involved in blood coagulation and said ligand is a compound having a molecular weight less than 3,000.

4. The method of claim 2 wherein said ligand is a divalent or trivalent metal cation.

5. The method of claim 4 wherein said cation is Ca⁺⁺, Mn⁺⁺, Co⁺⁺, Gd⁺⁺, or Mg⁺⁺.

6. The method of claim 4 wherein said higher binding affinity compound is a metal chelating agent.

7. The method of claim 2 wherein said protein contains γ-carboxyglutamic acid, said ligand is a divalent or trivalent metal cation, and said cation complexes with said protein at a binding site containing said γ-carboxyglutamic acid.

8. The method of claim 2 wherein said protein is one of the mammalian proteins Factor V, Factor VII, Factor VIII, Factor IX, Factor X, prothrombin, protein C, protein S, or serum albumin.

9. The method of claim 2 wherein said protein is an enzyme.

## Patentansprüche

1. Konformationsspezifischer, auf einem festen Trägermaterial immobilisierter Antikörper, 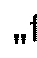 für ein freies Protein, das eine Konformationsänderung beim Komplexieren mit einem Liganden" erfährt, wobei dieser auf einem festen Trägermaterial immobilisierte Antikörper mit einem freien Protein, das mit einem Liganden komplexiert ist, reagieren kann und mit dem freien, nicht mit dem Liganden komplexierten Protein im wesentlichen reaktionsunfähig ist.

2. Verfahren zur Isolierung eines Proteins aus der Mischung, die das Protein enthält, wobei das Verfahren folgende Schritte beinhaltet:
Bereitstellen eines auf einem festen Trägermaterial immobilisierten konformationsspezifischen Antikörpers, wobei der Antikörper mit dem mit einem Liganden komplexierten Protein reagieren kann und mit dem nicht mit dem Liganden komplexierten Protein im wesentlichen nicht reaktionsfähig ist,
Kontaktieren der Mischung in Gegenwart des Liganden, mit dem immobilisierten Antikörper, um das mit dem Liganden komplexierte Protein an den immobilisierten Antikörper zu binden und einen Immunkomplex zu bilden und
Kontaktieren des Immunkomplexes mit einer Verbindung, die eine höhere Bindungsaffinität zu dem Liganden aufweist als das Protein, um das Protein von dem immobilisierten Antikörper freizusetzen.

3. Verfahren nach Anspruch 2, dadurch gekennzeichnet, daß das Protein bei der Blutgerinnung mitwirkt und daß der Ligand eine Verbindung mit einem Molekulargewicht unter 3.000 ist.

4. Verfahren nach Anspruch 2, dadurch gekennzeichnet, daß der Ligand ein zwei- oder dreiwertiges Metallkation ist.

5. Verfahren nach Anspruch 4, dadurch gekennzeichnet, daß das Kation Ca⁺⁺, Mn⁺⁺, Co⁺⁺, Gd⁺⁺ oder Mg⁺⁺ ist

6. Verfahren nach Anspruch 4, dadurch gekennzeichnet, daß die Verbindung mit der höheren Bindungsaffinität ein Metallchelatbildner ist.

7. Verfahren nach Anspruch 2, dadurch gekennzeichnet, daß das Protein γ-Carboxyglutaminsäure enthält, der Ligand ein zwei- oder dreiwertiges Metallkation ist, und das Kation an einer die γ-Carboxyglutaminsäure enthaltenden Bindungsstelle einen Komplex mit dem Protein bildet.

8. Verfahren nach Anspruch 2, dadurch gekennzeichnet, daß das Protein eines der Säugetierproteine Faktor V, Faktor VII, Faktor VIII, Faktor IX, Faktor X, Prothrombin, Protein C, Protein S oder Serumalbumin ist.

9. Verfahren nach Anspruch 2, dadurch gekennzeichnet, daß das Protein ein Enzym ist.

## Revendications

1. Anticorps spécifique d'une conformation, "dirigé contre une protéine libre qui subit des modifications conformationnelles lorsqu'elle est complexée avec un ligand", immobilisé sur un support solide, ledit anticorps immobilisé sur ledit support réagissant avec une protéine libre complexée avec un ligand et ne réagissant sensiblement pas avec ladite protéine libre non complexée avec ledit ligand.

2. Procédé d'isolement d'une protéine d'un mélange contenant ladite protéine, ledit procédé comprenant
la mise à disposition d'un anticorps spécifique d'une conformation, immobilisé sur un support solide, ledit anticorps réagissant avec ladite protéine complexée avec un ligand et ne réagissant sensiblement pas avec ladite protéine non complexée avec ledit ligand,
la mise en contact dudit mélange, en présence dudit ligand, avec ledit anticorps immobilisé pour fixer ladite protéine, complexée avec ledit ligand, audit anticorps immobilisé pour former un complexe immun et
la mise en contact dudit complexe immun avec un composé présentant une affinité de fixation pour ledit ligand supérieure à l'affinité de fixation de ladite protéine pour ledit ligand, pour libérer ladite protéine dudit anticorps immobilisé.

3. Procédé selon la revendication 2, dans lequel ladite protéine est une protéine impliquée dans la coagulation sanguine et ledit ligand est un composé ayant une masse moléculaire inférieure à 3 000.

4. Procédé selon la revendication 2, dans lequel ledit ligand est un cation métal divalent ou trivalent.

5. Procédé selon la revendication 4, dans lequel ledit cation est Ca⁺⁺, Mn⁺⁺, Co⁺⁺, Gd⁺⁺ ou Mg⁺⁺.

6. Procédé selon la revendication 4, dans lequel ledit composé à affinité de fixation supérieure est un agent chélateur de métal.

7. Procédé selon la revendication 2, dans lequel ladite protéine contient de l'acide γ-carboxyglutamique, ledit ligand est un cation métal divalent ou trivalent et ledit cation forme un complexe avec ladite protéine à un site de fixation contenant ledit acide γ-carboxyglutamique.

8. Procédé selon la revendication 2, dans lequel ladite protéine est une des protéines mammaliennes facteur V, facteur VII, facteur VIII, facteur IX, facteur X, prothrombine, protéine C, protéine S ou sérumalbumine.

9. Procédé selon la revendication 2, dans lequel ladite protéine est une enzyme.
